# EUROPEAN PATENT SPECIFICATION

(11) **EP 1 837 019 B1**
(45) Date of publication and mention of the grant of the patent: **29.08.2012**
(21) Application number: 05807929.4
(22) Date of filing: 24.10.2005
(51) Int. Cl.: A61K 31/592, A61K 31/593, A61K 33/06, A61K 9/20, A61K 31/00, A61K 33/10, A61K 47/26, A61K 47/38

(54) **ORALLY-DISPERSIBLE PHARMACEUTICAL COMPOSITIONS**
Oral dispergierbare pharmazeutische Zusammensetzungen
Compositions pharmaceutiques orodispersibles

(30) Priority: 25.10.2004 ES 200402560
(43) Date of publication of application: 26.09.2007
(73) Proprietor: ITALFARMACO, S.A., 28108 Alcobendas (ES)
(72) Inventor: ACEBRÓN FERNÁNDEZ, Álvaro, E-28108 Alcobendas (ES); BLANCO LOUSAME, Dolores, E-28108 Alcobendas (ES); GASCÓ GUERRERO, Cándida, E-28108 Alcobendas (US)
(74) Representative: Quinterno, Giuseppe
(86) International application number: PCT/ES2005/000566
(87) International publication number: WO 2006/045870

(56) References cited:
- WO-A2-2005/011639
- ES-A1- 2 192 136
- US-A- 5 965 162
- US-B1- 6 475 510
- "Rote Liste 2002" 2002, ECV EDITIO CANTOR VERLAG , AULENDORF , XP002528072 * paragraph [62003] * * paragraph [62042] *

## Description

The present invention relates to oral pharmaceutical compositions in the form of orally dispersible tablets containing calcium and optionally vitamin D and/or fluorine combined with pharmaceutically acceptable excipients useful for treatment or prevention of osteoporosis and other diseases characterized by loss of bone mass.

### STATE OF THE ART

The skeleton performs functions in support and protection of the soft organs as well as in mineral homeostasis and acid base homeostasis. To perform such functions bone possesses a complex anatomical-functional organization.

In bone composition a solid organic matrix (osteoid) formed by type I collagen is distinguished, which is strengthened with a deposit of mineral salts of calcium, magnesium, phosphate, carbonate, citrate, chlorine and fluorine. The most abundant salt is hydroxyapatite (Ca₁₀(PO₄)₆(OH)₂), which forms crystals interwoven with the collagen fibers.

In the bone of a normal adult the collagen fibers are preferentially arranged in layers, forming a lamellar structure. The bone lamellae have cavities that house cells (osteoclasts, osteoblasts and osteocytes) and are connected by Haversian canals through which interstitial liquids and blood circulate.

With respect to the structural organization two types of bone are recognized: cortical, compact and with thick layers of calcified osteoid; and trabecular, less dense and with numerous bone spikes.

Bone formation begins in the uterus and continues during infancy and adolescence to skeletal maturity. Over the remaining life, the bone is not a static element but is a very active tissue that is constantly renewed through a mechanism of remodeling. This mechanism consists of destruction of small amounts of bone carried out by osteoclasts (bone resorption) followed by replacement thereof by osteoblasts (bone formation). This activity is regulated both by systemic factors (parathyroid hormone, clacitonin, vitamin D) and local factors (cytokines, growth factors and peptides) and is affected by drugs, habits and various pathologies.

Osteoporosis is the most common alteration of the skeleton in which a reduction of bone mass is produced per unit volume with respect to what is considered normal for a specific age, sex and race, but the relation between organic and mineral phases is maintained. This relation can be altered in other metabolic bone diseases, like osteomalacia.

Osteoporosis can be of distinct types: primary, secondary, idiopathic and localized. Primary osteoporosis has as causal factors menopause (type I, characterized by loss of trabecular bone) and aging (type II, characterized by loss of cortical bone). Secondary osteoporosis can be caused by different diseases, certain drugs or prolonged immobilization. Idiopathic osteoporosis has an unknown cause.

Under normal conditions, bone loss produced by osteoclastic activity is restored by osteoblastic activity. However, in idiopathic osteoporosis, an imbalance between formation and resorption occurs either from an excess of activity of osteoclasts or a reduction in activity of osteoblasts. In type I osteoporosis, osteoclastic activity predominates and loss of bone mass is greater than under normal conditions, osteoblastic activity is normal but is not capable of compensating for the loss of bone mass. In type II osteoporosis, osteoclastic activity is normal but osteoblastic activity is reduced and is not capable of compensating for the loss.

Calcium is the predominant mineral in bone and is normally supplied to the body through the diet. However, in certain cases this supply is not sufficient and supplements of this mineral must be prescribed in order to prevent or treat osteoporosis and other diseases characterized by loss of bone mass.

The amount of recommended calcium varies as a function of the authors, age of the person and the administered salt. Generally the recommended amount for an adult is between 800 and 1500 mg/day.

The term vitamin D refers to a group of molecules with a steroid structure, including cholecalciferol (vitamin D3), ergocalciferol (vitamin D2), biologically active metabolites thereof (especially 1,25-dihydroxycholecalciferol) and precursors thereof (provitamin D2 present in foods of plant origin, provitamin D3 available in foods of animal origin).

This vitamin promotes intestinal absorption of calcium, phosphate and magnesium ions, contributes to regulation of calcium in the plasma by acting in the process of formation and resorption of bone, and stimulates resorption of calcium in the kidney. Overall the crucial effect of vitamin D on bone is to provide an appropriate balance of calcium and phosphorus to contribute to mineralization.

Vitamin D is produced in the body by the action of sun rays on precursors obtained from foods. However, in situations of deficient diet and/or rare exposure to sunlight, it will be necessary to administer supplements with vitamin D to prevent or treat osteoporosis and other bone diseases.

All the forms of vitamin D are liposoluble and accumulate in the body. They should not be administered in supraphysiological concentrations because they alter renal, neurological and digestive function. The recommended amounts of vitamin D vary between 200 and 800 IU/day, depending on the age and nutritional situation of the person.

Fluorine is mostly associated with calcified tissues (bones and teeth). It is a known inductor of osteoblastic proliferation. Normally it is supplied to the body through water and foods but in some cases it can be necessary to prescribe supplements, which are sodium fluoride (NaF) and sodium monofluorophosphate (MFP), the only clinically acceptable salts.

The optimum therapeutic range of fluorine ions is from 10 to 50 mg/day for an adult human. It is recommended that no more than 30 mg fluorine/day be administered, since it has been observed that when doses of 60 mg NaF/day are used, formation of abnormal bone is produced.

The use of compositions containing calcium and optionally vitamin D and/or fluorine in the prevention or treatment of osteoporosis is widely known.

In patent application WO 99/06051 pharmaceutical compositions containing calcium in the form of a salt, preferably calcium phosphate, vitamin D and a binder chosen from propylene glycol, polyethylene glycol with a molecular weight between 300 and 1500, liquid paraffin or silicone oil are disclosed.

In WO 96/09036 therapeutic combinations of vitamin D and a calcium salt, preferably calcium carbonate, are disclosed, which also contain a first binder in synergistic combination with a diluent, a second binder and a lubricant, the diluent and the second binder being a sweetener. The first binder is chosen from cellulose, maltodextrin and polyvinylpyrrolidone, the diluent is preferably xylitol, the second binder in the preferred form is sorbitol and the lubricant is generally magnesium stearate.

In WO 92/19251 nutritional substances containing calcium, specifically in the form of calcium malate-citrate salt, vitamin D and optionally an estrogen are disclosed.

In US 3345265 multilayer oral tablets with a central core of calcium salt and an external coating of fluoride, useful as a nutritional substance during pregnancy are disclosed.

ES-A1-2 192 136 discloses Chewable or effervescent tablets of calcium salts which contain different excipients.

However, the pharmaceutical formulations of calcium commercially available now have a number of problems that do not make them acceptable for everyone.

Tablets and capsules are the preferred pharmaceutical forms for administration of drugs because they can be precisely dosed, are easily produced on a large scale and contribute to good compliance with treatment.

However, some patients have difficulty in taking tablets and hard gelatin capsules and, as a result, do not take the medication as prescribed.

Orally dispersible tablets can be defined as solid forms that disintegrate or dissolve instantaneously or rapidly in the oral cavity without a need to administer liquids that form a solution or suspension that can be taken easily. They are also called fast melt tablets, quick dissolving tablets, orodispersible, bucodisintegrable, orodisintegrable or bucosoluble tablets.

These pharmaceutical forms are very useful in persons who suffer from dysphagia and have difficulty in swallowing tablets or traditional capsules, a fact that is particularly acute in the elderly and children. They are also useful in persons who do not have easy access to water, for example, vehicle drivers, attendees at long meetings, etc.

Orally dispersible tablets can be prepared by different methods, mostly freeze drying (lyophilization), formation by molding and direct compression.

The first two methods allow preparation of orally dispersible tablets that disintegrate in about 30 seconds but have low physical strength and high friability. On the other hand, direct compression provides tablets with greater friability but which disintegrate over a longer time.

### SUMMARY OF THE INVENTION

There is consequently a need to have tablets which:
- allow effective and non-toxic dosing of calcium and optionally vitamin D and/or fluorine, for treatment of osteoporosis and other diseases related to loss of bone mass,
- undergo rapid disintegration in contact with saliva and good palatability, improving the convenience and compliance by the patient at the same time as efficacy of treatment,
- exhibit pharmacotechnical characteristics such as adequate fluidity and easy compression of the components along with friability and adequate hardness of the final functional form,
- have a competitive cost and comparative advantages with other pharmaceutical forms.

In the first place, it is noted that tablets with a content of calcium salt between 45 and 90% by weight of the formulation are to be obtained in order to achieve the recommended daily ingestion of calcium administered in one or two tablets per day. But this active agent in increased percentage causes disagreeable palatability and limits the amount of excipients that can be incorporated in the formulation. The less acceptable taste and texture are therefore a problem that is difficult to solve in this type of formulation.

The inventors of the invention surprisingly found that the combination of large amounts of calcium salts with excipients with specific characteristics allows orally dispersible tablets to be obtained with an increased percentage of calcium salt and acceptable organoleptic properties.

On the other hand, it is noted that in general the effectiveness of disintegration is strongly affected by the size and hardness of the orally dispersible tablet. Optimal disintegration properties are often associated with medium or small sizes and/or low physical strength (high friability and low hardness).

As a result, a difficulty inherent to formulations in the form of orally dispersible tablets is to obtain adequate friability and hardness values as well as acceptable disintegration times.

According to European Pharmacopoeia 4.1, 2002 the disintegration time of an orally dispersible tablet must be less than 3 minutes.

The inventors of the present invention surprisingly found that, despite the limitation represented by the amount of excipients that can be incorporated in the composition, the use of certain excipients allows orally dispersible tablets to be obtained of a calcium salt and optionally vitamin D and/or fluorine salt by a direct compression method which satisfy the disintegration time required by the pharmacopoeia and at the same time have an adequate size, integrity and mechanical strength that allows them to be transported without fragility. At the same time orally dispersible tablets with adequate friability and hardness can be obtained.

To summarize, the excipients used in the present invention allow preparation of tablets with rapid disintegration in the mouth and at the same time acceptable taste and texture, which provides convenience to the patient and as a result improves compliance with treatment.

In addition, the orally dispersible tablets of the present invention, being obtained by direct compression, have a lower manufacturing cost than, for example, lyophilized tablets, since they use conventional production and packaging equipment, commonly available excipients and a limited number of steps.

At the same time, they enjoy clear advantages with respect to other pharmaceutical forms. In comparison with liquid forms, they allow administration of an exact dose of active principle and have better chemical stability and microbiological properties. Compared with chewable oral forms, they dissolve instantaneously in the oral cavity without requiring administration of liquids or chewing. In comparison with effervescent tablets, they have fewer production and preservation difficulties and at the same time do not require preparation prior to administration (they do not have to be dispersed beforehand in water) so that they enjoy better acceptance by the patient.

Additionally, the orally dispersible tablets allow the active principles to be immediately available to be absorbed and can do so before reaching the stomach through the oral, pharyngeal and esophageal mucosas.

As a result, the first aspect of the present invention concerns oral pharmaceutical compositions in the form of orally dispersible tablets, which contain elemental calcium and optionally vitamin D and/or fluorine as active principle(s), combined with pharmaceutically acceptable excipients.

The second aspect of the present invention concerns the use of the orally dispersible tablets of calcium and optionally vitamin D and/or fluorine in the prevention or treatment of osteoporosis and other diseases characterized by loss of bone mass.

A third aspect of the present invention concerns a method for preparation of the orally dispersible tablets of calcium and optionally vitamin D and/or fluorine by direct compression.

### DETAILED DESCRIPTION

The formulations of this invention preferably contain elemental calcium in an amount between 15 and 35 wt% of the formulation, at least one disintegrant and at least one sweetener.

In a preferred embodiment the orally dispersible tablets of the present invention contain:
- between 400 and 700 mg elemental calcium;
- at least one disintegrant;
- at least one sweetener.

In another preferred embodiment the orally dispersible tablets of the present invention contain:
- between 400 and 700 mg elemental calcium;
- between 100 and 500 IU vitamin D;
- at least one disintegrant;
- at least one sweetener.

In another preferred embodiment the orally dispersible tablets of the present invention contain:
- between 400 and 700 mg elemental calcium;
- between 100 and 500 IU vitamin D;
- between 2 and 30 mg fluorine;
- at least one disintegrant;
- at least one sweetener.

The elemental calcium is supplied in the form of a salt.

Preferably the calcium salt is chosen from calcium carbonate, calcium pidolate, calcium lactate, calcium citrate, calcium gluconate, calcium choride, calcium glucoheptonate, calcium glycerophosphate, calcium phosphate and mixtures thereof.

In a more preferred embodiment the calcium salt is chosen from the group formed by calcium carbonate, calcium gluconate, calcium lactate, calcium citrate and mixtures thereof.

In an even more preferred embodiment the calcium salt is calcium carbonate in a percentage between 45 and 90% by weight of the formulation, more preferably between 70 and 80%. In particular, tablets with a weight between 2000 and 2200 mg and with a content of calcium carbonate between 1000 and 1750 mg are preferred, more preferably between 1250 and 1750 mg. This salt has a double function as source of calcium and as an agent capable of releasing carbon dioxide (CO₂) in the presence of an acid at the same time that it furnishes a significant supply of elemental calcium by weight of the salt than other salts and has greater bioavailability.

Optionally the calcium salt can be pregranulated with maltodextrins or with pregelatinized starch, in order to obtain a raw material with adequate flow and compressibility characteristics.

Mixtures of calcium carbonate/maltodextrin with a weight ratio of 95/5 and 90/10 are preferably used.

Optionally the vitamin D can be stabilized with antioxidants.

Preferably vitamin D stabilized with DL-α-tocopherol was used.

The fluorine is supplied in the form of a salt.

Preferably the fluorine salt is chosen from sodium monofluorophosphate and sodium fluoride.

In a more preferred embodiment, the fluorine salt is sodium monofluorophosphate in an amount between 15 and 230 mg, more preferably between 50 and 200 mg.

The disintegrant is preferably chosen from crospovidon, sodium croscarmelose, guar gum, sodium glycolate of starch and cellulose derivatives, like hydroxypropylcellulose with a low degree of substitution.

In a more preferred embodiment the disintegrant is chosen from crospovidon, sodium croscarmelose, sodium glycolate of starch and hydroxypropylcellulose with a low degree of substitution, and is incorporated in an amount between 1 and 10 wt% of the formulation.

In an even more preferred embodiment the disintegrant is hydroxypropylcellulose with a low degree of substitution (L-HPC), added in an amount between 4 and 6 wt% of the formulation.

Preferably L-HPC is chosen from two varieties (LH-11 and LH-21) with different particle size, density and degree of substitution.

The sweetener is preferably a sweetener of intense flavor chosen from the group formed by aspartame, sodium saccharine, sodium cyclamate, potassium acesulfame and mixtures thereof, and is added in an amount between 0.1 and 1% by weight of the formulation.

In a more preferred embodiment the sweetener of intense flavor is selected from aspartame and mixtures containing it.

In an even more preferred embodiment the sweetener of intense flavor is aspartame added in an amount between 0.15 and 0.55% by weight of the formulation.

The sweetener of intense flavor can be optionally accompanied by at least one flavoring chosen from the group formed by oils of orange, lemon, strawberry, wild fruits, mint and anise in an amount between 0.01 and 1% by weight of the formulation.

The orally dispersible tablets of the present invention can also contain other pharmaceutically acceptable excipients.

In the preferred form an effervescent agent is present consisting of an agent capable of liberating CO₂ combined with an agent that induces liberation of CO₂.

The agent capable of liberating CO₂ can be selected from carbonates and bicarbonates of alkali metals.

In a particularly preferred embodiment the agent capable of liberating CO₂ is calcium carbonate.

The agent that induces liberation of CO₂ can be chosen from organic acids, acid salts thereof and mixtures thereof.

In a more preferred embodiment the agent that induces liberation of CO₂ is an organic acid selected from tartaric acid, malic acid, fumaric acid, succinic acid, ascorbic acid, maleic acid, citric acid, acid salts of said acids and mixtures thereof.

In an even more preferred embodiment the organic acid is chosen from anhydrous citric acid, tartaric acid, ascorbic acid and mixtures thereof. And more preferably it is anhydrous citric acid in an amount between 2 and 25 wt% of the formulation.

The agent that induces liberation of CO₂ is preferably in solid form and can be used in different particle sizes chosen between the powder and granular form.

In a particularly preferred embodiment, the effervescent agent consists of calcium carbonate/citric acid in a weight ratio between 10/1 and 2/1 but preferably between 8/1 and 4/1.

Preferably the tablets of the present invention will also contain at least one diluent-binder selected from the group formed by lactose, plasdon, maltodextrin, microcrystalline cellulose and dextrates in an amount between 1 and 10 wt% of the formulation.

In a particularly preferred embodiment the diluent is lactose incorporated in an amount between 1 and 5 wt% of the formulation.

Preferably the formulation will also contain at least one lubricant chosen from the group formed by stearic acid, magnesium stearate, sodium stearylfumarate, calcium stearate and polyethylene glycols in an amount between 0.5 and 5 wt% of the formulation.

In a particularly preferred embodiment the lubricant is stearic acid, added in an amount between 1 and 3 wt% of the formulation.

The tablets of the present invention can also contain at least one sweetener with a not very intense taste selected from the group formed by sorbitol, mannitol, xylitol, fructose, maltose, maltitol, lactitol and mixtures thereof in an amount between 1 and 20 wt% of the formulation.

Nevertheless, the tablets will preferably not contain a sweetener of mild flavor.

In a particularly preferred embodiment the orally dispersible tablets of the present invention contain:
- between 1250 mg and 1750 mg calcium carbonate;
- between 50 mg and 150 mg hydroxypropylcellulose with a low degree of substitution;
- between 100 mg and 450 mg citric acid;
- between 4 mg and 15 mg aspartame;
- optionally between 300 IU and 500 IU vitamin D;
- optionally between 50 mg and 200 mg monofluorophosphate.

Both the excipients and the active principles used in the tablets of the present invention are known and can be obtained from commercial sources.

The orally dispersible tablets of the present invention are useful in the treatment or prevention of osteoporosis and other diseases characterized by loss of bone mass.

### EXAMPLES

The invention is illustrated with the following nonlimiting examples:

### Example 1. Preparation processes

The tablets of the present invention are prepared following the direct comopression technique.

In a particular embodiment the sweetener, disintegrant and optionally vitamin D are mixed.

The premix is put into a mixer along with the calcium salt and optionally organic acid, flavoring, diluent-binder and/or lubricant. The obtained mixture is compressed in a rotary compression machine.

In another particular embodiment the calcium salt, disintegrant and optionally organic acid and/or diluent-binder are premixed. The premix is withdrawn from the mixer and a quarter of the weight thereof is introduced to the same mixer. The sweetener and optionally vitamin D and/or flavoring are incorporated in the form of a sandwich (in the interior) and mixed.

Another quarter of the premix is added and mixed. This procedure is repeated with each remaining quarter part of the premix. Lubricant is optionally added and mixed. Finally the resulting mixture is compressed in a rotary machine.

### Example 2. Formulations

The obtained orally dispersible tablets can have adequate technical characteristics and were well accepted in tests with healthy volunteers.

In the following detailed formulations, calcium carbonate 95% MD and 90% MD denotes calcium carbonate granulated with 5% and 10% by weight maltodextrin respectively; cholecalciferol represents vitamin D3 stabilized with DL-a-tocopherol in which 1 mg of cholecalciferol is equivalent to 100 IU active principle.

The tests to determine tensile strength, friability and disintegration time of the tablets were carried out according to the Royal Spanish Pharmacopoeia, 2^{nd} edition, 2002.

| Formula A | (mg) | (%) |
|---|---|---|
| Calcium carbonate 95% MD | 1578.95 | 73.34 |
| Granular citric acid | 394.74 | 18.34 |
| Lactose monohydrate | 64.73 | 3.01 |
| Sodium croscarmelose | 86.30 | 4.01 |
| Magnesium stearate | 21.58 | 1.00 |
| Aspartame | 4.32 | 0.20 |
| Orange oil | 2.16 | 0.10 |
| Tensile strength of the tablet (kgf) = 7.4 | | |
| Friability (%) = 3.54 | | |
| Disintegration time (min) = 1.8 | | |

| Formula B | (mg) | (%) |
|---|---|---|
| Cholecalciferol | 4.00 | 0.19 |
| Calcium carbonate 90% MD | 1666.00 | 79.26 |
| Anhydrous granular citric acid | 208.11 | 9.90 |
| L-HPC LH-11 | 105.08 | 5.00 |
| Lactose monohydrate | 63.05 | 3.00 |
| Stearic acid | 42.03 | 2.00 |
| Aspartame | 9.46 | 0.45 |
| Orange oil | 4.20 | 0.20 |
| Tensile strength of the tablet (kgf) = 8.7 | | |
| Friability (%) = 0.28 | | |
| Disintegration time (min) = 0.7 | | |

| Formula C | (mg) | (%) |
|---|---|---|
| Cholecalciferol | 4.00 | 0.19 |
| Calcium carbonate 95% MD | 1578.95 | 73.26 |
| Granular citric acid | 394.74 | 18.31 |
| Sodium croscarmelose | 86.06 | 4.00 |
| Lactose monohydrate | 64.54 | 3.00 |
| Magnesium stearate | 21.51 | 1.00 |
| Aspartame | 4.30 | 0.20 |
| Lemon oil | 1.29 | 0.06 |
| Tensile strength of the tablet (kgf) = 5.5 | | |
| Disintegration time (min) = 1.0-1.5 | | |

| Formula D | (mg) | (%) |
|---|---|---|
| Cholecalciferol | 4.00 | 0.19 |
| Calcium carbonate 95% MD | 1578.95 | 73.26 |
| Granular citric acid | 394.74 | 18.31 |
| Crospovidon | 86.06 | 4.00 |
| Lactose monohydrate | 64.54 | 3.00 |
| Stearic acid | 21.51 | 1.00 |
| Aspartame | 4.30 | 0.20 |
| Orange oil | 1.29 | 0.06 |
| Tensile strength of the tablet (kgf) = 7.3 | | |
| Friability (%) = 2.23 | | |
| Disintegration time (min) = 1.0-1.5 | | |

| Formula E | |
|---|---|
| Cholecalciferol | 4.00 |
| Calcium carbonate 95% MD | 1578.95 |
| Granular citric acid | 394.74 |
| Lactose monohydrate | 63.16 |
| Sodium starch glycolate | 42.11 |
| Magnesium stearate | 21.05 |
| Aspartame | 4.21 |
| Orange oil | 1.26 |
| Tensile strength of the tablet (kgf) = 6.2 | |
| Disintegration time (min) = 1.0-1.5 | |

| Formula F | |
|---|---|
| Cholecalciferol | 4.00 |
| Calcium carbonate 95% MD | 1578.95 |
| Granular citric acid | 394.74 |
| Mannitol | 244.45 |
| L-HPC LH-21 | 122.23 |
| Lactose monohydrate | 73.34 |
| Magnesium stearate | 24.45 |
| Aspartame | 4.89 |
| Strawberry oil | 1.47 |
| Tensile strength of the tablet (kgf) = 8.7 | |
| Disintegration time (min) = 1.0-1.8 | |

| Formula G | |
|---|---|
| Cholecalciferol | 4.00 |
| Calcium carbonate 95% MD | 1578.95 |
| Granular citric acid | 394.74 |
| L-HPC LH-21 | 109.07 |
| Lactose monohydrate | 65.44 |
| Stearic acid | 21.82 |
| Aspartame | 4.36 |
| Orange oil | 2.18 |
| Tensile strength of the tablet (kgf) = 8.7 | |
| Friability (%) = 2.75 | |
| Disintegration time (min) = 1.4 | |

| Formula H | |
|---|---|
| Cholecalciferol | 4.00 |
| Calcium carbonate 95% MD | 1578.95 |
| Anhydrous granular citric acid | 394.74 |
| Guar gum | 86.06 |
| Lactose monohydrate | 64.54 |
| Magnesium stearate | 21.51 |
| Aspartame | 4.30 |
| Orange oil | 1.29 |
| Tensile strength of the tablet (kgf) = 5.8 | |
| Disintegration time (min) = 1.0-1.5 | |

| Formula I | |
|---|---|
| Cholecalciferol | 4.00 |
| Calcium carbonate 95% MD | 1578.95 |
| Anhydrous granular citric acid | 394.74 |
| L-HPC LH-21 | 113.77 |
| Sodium croscarmelose | 91.02 |
| Lactose monohydrate | 68.26 |
| Stearic acid | 22.75 |
| Aspartame | 4.55 |
| Orange oil | 1.37 |
| Tensile strength of the tablet (kgf) = 7.0 | |
| Disintegration time (min) = 1.5-2.0 | |

| Formula J | |
|---|---|
| Cholecalciferol | 4.00 |
| Calcium carbonate 95% MD | 1676.91 |
| Granular citric acid | 209.62 |
| Granular tartaric acid | 209.62 |
| L-HPC LH-21 | 116.27 |
| Lactose monohydrate | 69.76 |
| Stearic acid | 23.25 |
| Aspartame | 10.46 |
| Orange oil | 4.65 |
| Tensile strength of the tablet (kgf) = 8.0 | |
| Friability (%) = 1.64 | |
| Disintegration time (min) = 0.9 | |

| Formula K | |
|---|---|
| Cholecalciferol | 4.00 |
| Calcium carbonate 95% MD | 1578.95 |
| Anhydrous granular citric acid | 394.74 |
| L-HPC LH-11 | 109.81 |
| Lactose monohydrate | 65.88 |
| Stearic acid | 21.96 |
| Aspartame | 5.00 |
| Sodium saccharine | 5.00 |
| Orange oil | 10.00 |
| Tensile strength of the tablet (kgf) = 8.0 | |
| Friability (%) = 2.61 | |
| Disintegration time (min) = 1.3 | |

| Formula L | |
|---|---|
| Cholecalciferol | 4.00 |
| Calcium carbonate 95% MD | 1578.95 |
| Anhydrous granular citric acid | 197.37 |
| Granular ascorbic acid | 197.37 |
| L-HPC LH-11 | 109.53 |
| Lactose monohydrate | 65.72 |
| Stearic acid | 21.91 |
| Aspartame | 10.00 |
| Orange oil | 5.00 |
| Tensile strength of the tablet (kgf) = 8.7 | |
| Friability (%) = 2.51 | |
| Disintegration time (min) = 1.7 | |

| Formula M | (mg) | (%) |
|---|---|---|
| Cholecalciferol | 4.00 | 0.19 |
| Calcium carbonate 90% MD | 1666.67 | 79.11 |
| Sodium monofluorophosphate | 100.00 | 4.75 |
| Citric acid | 200.00 | 9.49 |
| L-HPC LH-11 | 100.00 | 4.75 |
| Magnesium stearate | 24.00 | 1.14 |
| Aspartame | 10.00 | 0.47 |
| Oil of wild fruits | 2.50 | 0.12 |

## Claims

1. An orally dispersible pharmaceutical composition having a disintegration time less than three minutes, comprising:
- elemental calcium in an amount between 15% and 35% by weight of the composition;
- at least one disintegrant selected from the group consisting of crospovidon, sodium starch glycolate, sodium croscarmelose, and low-substituted hydroxypropyl-cellulose in an amount between 1% and 10% by weight of the composition;
- an effervescent agent, consisting of at least one agent to liberate CO₂ selected from the group consisting of alkali metal carbonates and alkali metal bicarbonates, combined with at least one agent that induces liberation of CO₂ selected from the group consisting of organic acids, acid salts of organic acids and mixtures thereof;
- a diluent-binder selected from the group consisting of lactose, plasdone, maltodextrin, microcrystalline cellulose and dextrates, in an amount between 1% and 10% by weight of the composition; and
- at least one sweetener of intense flavour;

2. The composition according to claim 1 wherein the elemental calcium is between 400 mg and 700 mg.

3. The composition according to claim 1 or 2 wherein the elemental calcium is derived from a calcium salt selected from the group consisting of calcium carbonate, calcium pidolate, calcium lactate, calcium citrate, calcium gluconate, calcium chloride, calcium glucoheptonate, calcium phosphate, calcium glycerophosphate and mixtures thereof.

4. The composition according to claim 3 wherein said calcium salt is selected from the group consisting of calcium carbonate, calcium gluconate, calcium lactate, calcium citrate and mixtures thereof.

5. The composition according to claim 4 wherein said calcium salt is calcium carbonate in an amount between 45% and 90% by weight of the composition.

6. The composition according to claim 5 wherein said calcium carbonate is present in an amount between 1000 and 1750 mg.

7. The composition according to claims 3 to 6 wherein said calcium carbonate is pregranulated with a binder selected from the group consisting of maltodextrins and pregelatinized starch.

8. The composition according to claim 1 wherein said disintegrant is hydroxy-propylcellulose with a low degree of substitution in an amount between 4% and 6% by weight of the composition.

9. The composition according to claim 1 wherein the sweetener with an intense flavour is selected from the group consisting of aspartame, sodium saccharine, sodium cyclamate, potassium acesulfame and mixtures thereof, in an amount between 0.1% and 1% by weight of the composition.

10. The composition according to claim 9 wherein said sweetener is aspartame in an amount between 0.15% and 0.55% by weight of the composition.

11. The composition according to claim 1 wherein said agent to liberate CO₂ is calcium carbonate.

12. The composition according to claim 1 wherein the agent that induces liberation of CO₂ is selected from a group consisting of tartaric acid, malic acid, fumaric acid, succinic acid, ascorbic acid, maleic acid, citric acid, salts of said acids, and mixtures thereof.

13. The composition according to claim 12 wherein said agent that induces liberation of CO₂ is anhydrous citric acid in an amount between 2% and 25% by weight of the composition.

14. The composition according to claim 1 wherein the diluent-binder is lactose in an amount between 1% and 5% by weight of the composition.

15. The composition according to any of the preceding claims further comprising between 100 IU and 500 IU of vitamin D.

16. The composition according to any of the preceding claims further comprising between 2 mg and 30 mg of fluorine.

17. The composition according to claim 1 to 14 comprising:
- between 1250 and 1750 mg of calcium carbonate;
- between 50 mg and 150 mg of low-substituted hydroxypropylcellulose;
- between 100 mg and 450 mg of citric acid;
and
- between 4 mg and 15 mg of aspartame.

18. The composition according to claim 16 further comprising between 300 IU and 500 IU of vitamin D.

19. The composition according to claim 16 or 17 further comprising between 50 mg and 200 mg of sodium monofluorophosphate.

20. The composition according to any of the preceding claims further comprising one or more flavouring agent selected from the group consisting of (essential) oils of orange, lemon, strawberry, forest fruits, mint and anise, in an amount between 0.01% and 1% by weight of the composition.

21. The composition according to any of the preceding claims further comprising a lubricant selected from the group consisting of stearic acid, magnesium stearate, sodium stearyl fumarate, calcium stearate and polyethylene glycols, in an amount between 0.5% and 5% by weight of the composition.

22. Use of a composition according to any one of the preceding claims in the preparation of a drug for treatment or prevention of osteoporosis and other diseases entailing loss of bone mass.

23. A method for preparing a pharmaceutical composition according to claims 1 to 21, comprising:
- premixing the sweetener, the disintegrant, and optionally vitamin D, so as to form a premix;
- mixing the previous premix with the calcium salt, the organic acid, the diluent-binder, and optionally the flavouring agent and/or the lubricant, so as to form a mixture; and
- compressing the resulting mixture.

24. A method for preparing a pharmaceutical composition according to claims 1 to 21, comprising:
a) premixing the calcium salt, the disintegrant, the organic acid and the diluent-binder, in a mixer so as to form a premix;
b) removing the premix from the mixer;
c) adding a portion of the premix into the mixer;
d) adding the sweetener, and optionally vitamin D and/or the flavouring agent, in sandwich form into said portion of the premix, and mixing so as to form a mixture;
e) adding another portion of the premix and mixing;
f) repeating step e) using remaining portion(s) of the premix;
g) optionally adding the lubricant into the mixture and mixing so as to form a final mixture; and
h) compressing the resulting mixture.

## Patentansprüche

1. Oral dispergierbare pharmazeutische Zusammensetzung mit einer Zerfallszeit von weniger als drei Minuten, umfassend:
- elementares Calcium in einer Menge zwischen 15 Gew.% und 35 Gew.% der Zusammensetzung,
- mindestens ein Sprengmittel ausgewählt aus der Gruppe bestehend aus Crospovidon, Natrium-Stärkeglykolat, Natrium-Croscarmelose und niedrig substituierter Hydroxypropylcellulose in einer Menge zwischen 1 Gew.% und 10 Gew.% der Zusammensetzung,
- ein Brausemittel, das aus mindestens einem Mittel zur Freisetzung von CO₂ ausgewählt aus der Gruppe bestehend aus Alkalimetallcarbonaten und Alkalimetallbicarbonaten besteht, in Kombination mit mindestens einem Mittel, das die Freisetzung von CO₂ induziert, ausgewählt aus der Gruppe bestehend aus organischen Säuren, sauren Salzen von organischen Säuren und Mischungen davon,
- ein Verdünnungsmittel-Bindemittel ausgewählt aus der Gruppe bestehend aus Lactose, Plasdon, Maltodextrin, mikrokristalliner Cellulose und Dextraten in einer Menge zwischen 1 Gew.% und 10 Gew.% der Zusammensetzung, und
- mindestens ein Süßungsmittel mit intensivem Geschmack.

2. Zusammensetzung nach Anspruch 1, wobei das elementare Calcium zwischen 400 mg und 700 mg liegt.

3. Zusammensetzung nach Anspruch 1 oder 2, wobei das elementare Calcium von einem Calciumsalz ausgewählt aus der Gruppe bestehend aus Calciumcarbonat, Calciumpidolat, Calciumlactat, Calciumcitrat, Calciumgluconat, Calciumchlorid, Calciumglucoheptonat, Calciumphosphat, Calciumglycerophosphat und Mischungen davon abgeleitet ist.

4. Zusammensetzung nach Anspruch 3, wobei das Calciumsalz ausgewählt ist aus der Gruppe bestehend aus Calciumcarbonat, Calciumgluconat, Calciumlactat, Calciumcitrat und Mischungen davon.

5. Zusammensetzung nach Anspruch 4, wobei das Calciumsalz Calciumcarbonat in einer Menge zwischen 45 Gew.% und 90 Gew.% der Zusammensetzung ist.

6. Zusammensetzung nach Anspruch 5, wobei das Calciumcarbonat in einer Menge zwischen 1000 und 1750 mg vorhanden ist.

7. Zusammensetzung nach den Ansprüchen 3 bis 6, wobei das Calciumcarbonat mit einem Bindemittel ausgewählt aus der Gruppe bestehend aus Maltodextrinen und Quellstärke vorgranuliert ist.

8. Zusammensetzung nach Anspruch 1, wobei das Sprengmittel Hydroxypropylcellulose mit einem niedrigen Substitutionsgrad in einer Menge zwischen 4 Gew.% und 6 Gew.% der Zusammensetzung ist.

9. Zusammensetzung nach Anspruch 1, wobei das Süßungsmittel mit einem intensiven Geschmack ausgewählt ist aus der Gruppe bestehend aus Aspartam, Saccharin-Natrium, Natriumcyclamat, Kalium-Acesulfam und Mischungen davon in einer Menge zwischen 0,1 Gew.% und 1 Gew.% der Zusammensetzung.

10. Zusammensetzung nach Anspruch 9, wobei das Süßungsmittel Aspartam in einer Menge zwischen 0,15 Gew.% und 0,55 Gew.% der Zusammensetzung ist.

11. Zusammensetzung nach Anspruch 1, wobei das Mittel zur Freisetzung von CO₂ Calciumcarbonat ist.

12. Zusammensetzung nach Anspruch 1, wobei das Mittel, das die Freisetzung von CO₂ induziert, ausgewählt ist aus einer Gruppe bestehend aus Weinsäure, Apfelsäure, Fumarsäure, Bernsteinsäure, Ascorbinsäure, Maleinsäure, Citronensäure, Salzen der Säuren sowie Mischungen davon.

13. Zusammensetzung nach Anspruch 12, wobei das Mittel, das die Freisetzung von CO₂ induziert, wasserfreie Citronensäure in einer Menge zwischen 2 Gew.% und 25 Gew.% der Zusammensetzung ist.

14. Zusammensetzung nach Anspruch 1, wobei das Verdünnungsmittel-Bindemittel Lactose in einer Menge zwischen 1 Gew.% und 5 Gew.% der Zusammensetzung ist.

15. Zusammensetzung nach einem der vorhergehenden Ansprüche, die ferner zwischen 100 IE und 500 IE Vitamin D umfasst.

16. Zusammensetzung nach einem der vorhergehenden Ansprüche, die ferner zwischen 2 mg und 30 mg Fluor umfasst.

17. Zusammensetzung nach den Ansprüchen 1 bis 14, umfassend:
- zwischen 1250 und 1750 mg Calciumcarbonat,
- zwischen 50 mg und 150 mg niedrig substituierte Hydroxypropylcellulose,
- zwischen 100 mg und 450 mg Citronensäure und
- zwischen 4 mg und 15 mg Aspartam.

18. Zusammensetzung nach Anspruch 16, die ferner zwischen 300 IE und 500 IE Vitamin D umfasst.

19. Zusammensetzung nach Anspruch 16 oder 17, die ferner zwischen 50 mg und 200 mg Natriummonofluorphosphat umfasst.

20. Zusammensetzung nach einem der vorhergehenden Ansprüche, die ferner ein oder mehrere Aromatisierungsmittel ausgewählt aus der Gruppe bestehend aus (etherischen) Ölen von Orange, Zitrone, Erdbeer, Waldfrüchten, Minze und Anis in einer Menge zwischen 0,01 und 1 Gew.% der Zusammensetzung umfasst.

21. Zusammensetzung nach einem der vorhergehenden Ansprüche, die ferner ein Schmiermittel ausgewählt aus der Gruppe bestehend aus Stearinsäure, Magnesiumstearat, Natriumstearylfumarat, Calciumstearat und Polyethylenglykolen in einer Menge zwischen 0,5 und 5 Gew.% der Zusammensetzung umfasst.

22. Verwendung einer Zusammensetzung nach einem der vorhergehenden Ansprüche zur Herstellung eines Arzneimittels zur Behandlung oder Vorbeugung von Osteoporose und anderen Erkrankungen, die Verlust an Knochenmasse mit sich bringen.

23. Verfahren zur Herstellung einer pharmazeutischen Zusammensetzung nach den Ansprüchen 1 bis 21, umfassend:
- Vormischen des Süßungsmittels, des Sprengmittels und gegebenenfalls des Vitamins D, um eine Vormischung zu bilden,
- Mischen der vorhergehenden Vormischung mit dem Calciumsalz, der organischen Säure, dem Verdünnungsmittel-Bindemittel und gegebenenfalls dem Aromatisierungsmittel und/oder dem Schmiermittel, um eine Mischung zu bilden, und
- Komprimieren der resultierenden Mischung.

24. Verfahren zur Herstellung einer pharmazeutischen Zusammensetzung nach den Ansprüchen 1 bis 21, umfassend:
a) Vormischen des Calciumsalzes, des Sprengmittels, der organischen Säure und des Verdünnungsmittels-Bindemittels in einem Mischer, um eine Vormischung zu bilden,
b) Entfernen der Vormischen aus dem Mischer,
c) Zufügen einer Portion der Vormischung in den Mischer,
d) Zufügen des Süßungsmittels und gegebenenfalls des Vitamins D und/oder des Aromatisierungsmittels in Sandwichform in die Portion der Vormischung, und Mischen, um eine Mischung zu bilden,
e) Zufügen einer weiteren Portion der Vormischung, und Mischen,
f) Wiederholen von Schritt e) unter Verwendung von einer oder mehreren restlichen Portionen der Vormischung,
g) gegebenenfalls Zufügen des Schmiermittels zu der Mischung, und Mischen, um eine endgültige Mischung zu bilden, und
h) Komprimieren der resultierenden Mischung.

## Revendications

1. Composition pharmaceutique dispersible par voie orale ayant un temps de désintégration inférieur à trois minutes, comprenant :
- du calcium élémentaire en une quantité comprise entre 15 % et 35 % en poids de la composition ;
- au moins un délitant choisi dans le groupe constitué par la crospovidone, le glycolate d'amidon sodique, la croscarmellose sodique et l'hydroxypropylcellulose à faible substitution en une quantité comprise entre 1 % et 10 % en poids de la composition ;
- un agent effervescent, constitué d'au moins un agent pour libérer du CO₂ choisi dans le groupe constitué par les carbonates de métaux alcalins et les bicarbonates de métaux alcalins, combiné avec au moins un agent qui induit la libération de CO₂ choisi dans le groupe constitué par les acides organiques, les sels acides d'acides organiques et leurs mélanges ;
- un diluant-liant choisi dans le groupe constitué par le lactose, la plasdone, la maltodextrine, la cellulose microcristalline et les dextrates, en une quantité comprise entre 1 % et 10 % en poids de la composition ; et
- au moins un édulcorant de goût intense.

2. Composition selon la revendication 1, dans laquelle le calcium élémentaire est compris entre 400 mg et 700 mg.

3. Composition selon la revendication 1 ou 2, dans laquelle le calcium élémentaire est dérivé d'un sel de calcium choisi dans le groupe constitué par le carbonate de calcium, le pidolate de calcium, le lactate de calcium, le citrate de calcium, le gluconate de calcium, le chlorure de calcium, le glucoheptonate de calcium, le phosphate de calcium, le glycérophosphate de calcium et leurs mélanges.

4. Composition selon la revendication 3, dans laquelle ledit sel de calcium est choisi dans le groupe constitué par le carbonate de calcium, le gluconate de calcium, le lactate de calcium, le citrate de calcium et leurs mélanges.

5. Composition selon la revendication 4, dans laquelle ledit sel de calcium est le carbonate de calcium en une quantité comprise entre 45 % et 90 % en poids de la composition.

6. Composition selon la revendication 5, dans laquelle ledit carbonate de calcium est présent en une quantité comprise entre 1 000 et 1 750 mg.

7. Composition selon les revendications 3 à 6, dans laquelle ledit carbonate de calcium est pré-granulé avec un liant choisi dans le groupe constitué par les maltodextrines et l'amidon pré-gélatinisé.

8. Composition selon la revendication 1, dans laquelle ledit délitant est l'hydroxypropylcellulose ayant un faible degré de substitution en une quantité comprise entre 4 % et 6 % en poids de la composition.

9. Composition selon la revendication 1, dans laquelle l'édulcorant de goût intense est choisi dans le groupe constitué par l'aspartame, la saccharine de sodium, le cyclamate de sodium, l'acésulfame de sodium et leurs mélanges, en une quantité comprise entre 0,1 % et 1 % en poids de la composition.

10. Composition selon la revendication 9, dans laquelle ledit édulcorant est l'aspartame en une quantité comprise entre 0,15 % et 0,55 % en poids de la composition.

11. Composition selon la revendication 1, dans laquelle ledit agent pour libérer du CO₂ est le carbonate de calcium.

12. Composition selon la revendication 1, dans laquelle l'agent qui induit la libération de CO₂ est choisi dans un groupe constitué par l'acide tartrique, l'acide malique, l'acide fumarique, l'acide succinique, l'acide ascorbique, l'acide maléique, l'acide citrique, les sels desdits acides et leurs mélanges.

13. Composition selon la revendication 12, dans laquelle ledit agent qui induit la libération de CO₂ est l'acide citrique anhydre en une quantité comprise entre 2 % et 25 % en poids de la composition.

14. Composition selon la revendication 1, dans laquelle le diluant-liant est le lactose en une quantité comprise entre 1 % et 5 % en poids de la composition.

15. Composition selon l'une quelconque des revendications précédentes, comprenant également entre 100 UI et 500 UI de vitamine D.

16. Composition selon l'une quelconque des revendications précédentes, comprenant également entre 2 mg et 30 mg de fluor.

17. Composition selon l'une quelconque des revendications 1 à 14, comprenant :
- entre 1 250 et 1 750 mg de carbonate de calcium ;
- entre 50 mg et 150 mg d'hydroxypropylcellulose à faible substitution ;
- entre 100 mg et 450 mg d'acide citrique ; et
- entre 4 mg et 15 mg d'aspartame.

18. Composition selon la revendication 16, comprenant également entre 300 UI et 500 UI de vitamine D.

19. Composition selon la revendication 16 ou 17, comprenant également entre 50 mg et 200 mg de monofluorophosphate de sodium.

20. Composition selon l'une quelconque des revendications précédentes, comprenant également un ou plusieurs aromatisants choisis dans le groupe constitué par les huiles (essentielles) d'orange, de citron, de fraise, les fruits de la forêt, la menthe et l'anis, en une quantité comprise entre 0,01 % et 1 % en poids de la composition.

21. Composition selon l'une quelconque des revendications précédentes, comprenant également un lubrifiant choisi dans le groupe constitué par l'acide stéarique, le stéarate de magnésium, le fumarate de stéaryle sodique, le stéarate de calcium et les polyéthylène glycols, en une quantité comprise entre 0,5 % et 5 % en poids de la composition.

22. Utilisation d'une composition selon l'une quelconque des revendications précédentes pour la préparation d'un médicament pour le traitement ou la prévention de l'ostéoporose ou d'autres maladies impliquant une perte de masse osseuse.

23. Procédé de préparation d'une composition pharmaceutique selon les revendications 1 à 21, comprenant :
- le pré-mélange de l'édulcorant, du délitant et éventuellement de la vitamine D, afin de former un pré-mélange ;
- le mélange du pré-mélange précédent avec le sel de calcium, l'acide organique, le diluant-liant et éventuellement l'aromatisant et/ou le lubrifiant, afin de former un mélange ; et
- la compression du mélange résultant.

24. Procédé de préparation d'une composition pharmaceutique selon les revendications 1 à 21, comprenant :
a) le pré-mélange du sel de calcium, du délitant, de l'acide organique et du diluant-liant dans un mélangeur afin de former un pré-mélange ;
b) le déchargement du pré-mélange du mélangeur ;
c) l'ajout d'une partie du pré-mélange dans le mélangeur ;
d) l'ajout de l'édulcorant et éventuellement de la vitamine D et/ou de l'aromatisant, sous la forme d'un sandwich dans ladite partie du pré-mélange, et le mélange afin de former un mélange ;
e) l'ajout d'une autre partie du pré-mélange et le mélange ;
f) la répétition de l'étape e) en utilisant la ou les parties restantes du pré-mélange ;
g) éventuellement l'ajout du lubrifiant dans le mélange et le mélange afin de former un mélange final ; et
h) la compression du mélange résultant.
